# FASCICULE DE BREVET EUROPEEN

(11) **EP 2 263 747 B1**
(45) Date de publication et mention de la délivrance du brevet: **26.08.2015**
(21) Numéro de dépôt: 10162507.7
(22) Date de dépôt: 11.05.2010
(51) Int. Cl.: A61N 1/365

(54) **Prothèse cardiaque implantable comprenant des moyens d'analyse de la tolérance du patient à un mode de stimulation privilégiant la conduction atrio-ventriculaire spontanée**
Implantierbare Herzprothese mit Mittel zur Analyse der Patiententoleranz bezüglich der Stimulationsart die spontane Atrio-Ventrukuläre Leitung bevorzugend
Implantable cardiac prosthesis comprising means for analysing patient's tolerance of stimulation mode aiming at spontaneous atrio-ventricular conduction

(30) Priorité: 15.06.2009 FR 0953982
(43) Date de publication de la demande: 22.12.2010
(73) Titulaire: Sorin CRM SAS, 92541 Montrouge (FR)
(72) Inventeur: Graindorge, Laurence, 44470 Thouaré sur Loire (FR)
(74) Mandataire: Dupuis-Latour, Dominique

(56) Documents cités:
- EP-A1- 0 488 904
- EP-A1- 1 059 099
- EP-A1- 1 346 750
- US-A- 5 549 650
- US-A1- 2005 240 235
- US-A1- 2006 293 715

## Description

L'invention concerne les "dispositifs médicaux implantables actifs" tels que définis par la directive 90/385/CEE du 20 juin 1990 du Conseil des communautés européennes, et plus précisément les dispositifs stimulateurs cardiaques, dispositifs "multisite" (triple ou quadruple chambre), défibrillateurs et/ou cardioverteurs, permettant de surveiller l'activité cardiaque et de délivrer au coeur des impulsions électriques de stimulation, de resynchronisation, de cardioversion et/ou de défibrillation en cas de trouble du rythme diagnostiqué par l'appareil.

Elle concerne plus particulièrement ceux de ces dispositifs qui comprennent des circuits de stimulation et de détection à la fois sur l'oreillette et sur le ventricule. Le mode de fonctionnement de base d'un tel dispositif est un mode AAI - ou plus précisément "pseudo-AAI" - avec stimulation auriculaire simple chambre (mode AAI *stricto sensu*) et surveillance de l'activité ventriculaire. Ce mode est en principe maintenu tant que la conduction auriculo-ventriculaire est normale, c'est-à-dire tant que chaque événement auriculaire (détection auriculaire, correspondant à une activité spontanée, ou stimulation auriculaire) est suivi d'une détection ventriculaire associée.

En revanche, en fonctionnement "double chambre", après un événement auriculaire - qu'il s'agisse d'une dépolarisation spontanée (onde P détectée) ou stimulée (application d'une impulsion A) - le dispositif surveille l'activité ventriculaire et, en même temps, commence à compter un délai dit "délai atrio-ventriculaire" ou "délai auriculo-ventriculaire", généralement désigné DAV ou AVD. Si, à l'issue de ce délai aucune activité spontanée ventriculaire (onde R) n'a été détectée, alors le dispositif déclenche une stimulation de ce ventricule (application d'une impulsion V).

Les études cliniques récentes ont démontré que, pour prévenir l'occurrence d'une insuffisance cardiaque ou d'une fibrillation auriculaire chez les patients porteurs de stimulateurs cardiaques double chambre, il est recommandé d'éviter au maximum la stimulation ventriculaire droite lorsque cela est possible, de manière à préserver la conduction auriculo-ventriculaire intrinsèque chez les patients n'ayant par ailleurs pas de trouble permanent de la conduction imposant le recours à une stimulation ventriculaire définitive.

À cet égard, les nouveaux modes de stimulation des dispositifs récents permettent de privilégier la conduction ventriculaire spontanée, en autorisant des fonctionnements avec des DAV très longs permettant à la conduction spontanée de s'exprimer - mais avec en contrepartie le risque d'accepter le maintien de DAV longs symptomatiques.

Certains stimulateurs cardiaques, par exemple du type décrit dans le EP 0 488 904 A (ELA Medical) sont équipés d'algorithmes d'hystérésis du DAV, qui sont utilisables chez des patients stimulés en mode DDD mais n'ayant pas de trouble permanent de la conduction. Ces dispositifs peuvent opérer selon deux modes de fonctionnement, DDD ou AAI (le mode AAI étant en fait un mode DDD modifié par allongement du délai auriculo-ventriculaire), et sont pourvus d'un mode dénommé "DDD-CAM" assurant une commutation automatique de mode (CAM) de DDD en AAI et inversement. La valeur du DAV n'est pas fixe, mais varie linéairement entre une valeur maximale, utilisée lorsque la fréquence cardiaque est proche de la fréquence au repos, et une valeur minimale, lorsque la fréquence cardiaque est proche de sa valeur maximale. (le détail de cette adaptation du DAV est décrit par exemple dans le EP 1 059 099 A (ELA Medical)). L'idée de base de l'algorithme d'hystérésis du DAV est de prolonger la valeur du DAV sous certains critères, de manière à permettre au rythme spontané de s'exprimer : le DAV peut ainsi être allongé pour éviter des stimulations ventriculaires inutiles ou bien, au contraire, être peu à peu raccourci si aucun rythme spontané n'est découvert, jusqu'à revenir à une valeur habituelle de stimulation DDD classique.

Cette technique permet de préserver la conduction naturelle chez certains patients, mais s'avère d'une utilité limitée chez d'autres (notamment ceux en dysfonction sinusale), car les valeurs limites de l'hystérésis ne permettent pas de découvrir le rythme spontané.

Pour pallier cette limitation, un nouveau mode de stimulation a été développer dans des dispositifs plus récents, mode dénommé "AAISafeR", et dont le principe de base est exposé dans le EP 1 346 750 A (ELA Medical).

Dans ce mode de fonctionnement, le stimulateur fonctionne en mode AAI, et l'activité ventriculaire est surveillée en permanence de manière à détecter l'apparition de blocs auriculo-ventriculaires (BAV) entraînant un trouble temporaire de dépolarisation du ventricule. Dans ce cas, dès lors qu'un certain nombre de conditions sont remplies, le stimulateur bascule automatiquement en mode DDD automatique, avec des paramètres optimisés pour cette situation de BAV temporaire. Dans un appareil multisite, la commutation s'opèrera vers un mode de stimulation biventriculaire, dénommé "AAISafeRR".

Après disparition du BAV, et donc rétablissement de la conduction auriculo-ventriculaire, le stimulateur retourne automatiquement au mode AAI dès lors qu'un certain nombre d'autres conditions sont remplies. Le mode AAISafeR (ou AAISafeRR) prévoit également un critère de commutation lié à la détection d'un BAV du premier type ou BAV1, c'est-à-dire la présence d'un DAV trop long : lorsque l'appareil détecte par exemple plus de six cycles consécutifs avec un DAV trop long (typiquement, supérieurs à 350-450 ms), alors l'appareil opère une commutation en mode DDD, ou de la même manière en mode biventriculaire sur un appareil multisite.

Le mode AAISafeR (ou AAISafeRR) permet de préserver la conduction naturelle de façon très efficace, et les résultats cliniques montrent un pourcentage de stimulations ventriculaires résiduelles proche de zéro. Divers perfectionnements y ont été apportés, par exemple pour éliminer l'incidence des extrasystoles ventriculaires (cf. le EP 1 470 836 A (ELA Medical)) et/ou les BAV paroxystiques (cf. le EP 1 550 480 A (ELA Medical)), ou en présence d'événements ventriculaires de nature incertaine survenant en fenêtre de sécurité (cf. le EP 1 731 195 A (ELA Medical)), ou encore en présence de tachycardies ventriculaires (cf. le EP 1 731 194 A (ELA Medical)).

L'une des particularités du mode de stimulation AAISafeR (ou AAISafeRR) est d'autoriser des DAV très longs, dont la limite est éventuellement programmable. Mais de tels délais peuvent être plus ou moins bien supportés par le patient, ou peuvent être bien supportés dans certaines conditions (en particulier au repos) et moins bien dans d'autres (notamment à l'effort). Chez des patients souffrant de brady-tachycardies et prenant des anti-arythmiques, on a notamment rapporté l'apparition de symptômes liés à des DAV trop longs en fonctionnement AAI.

Chez certains patients, il faut donc mettre en balance le risque de stimuler trop souvent le ventricule droit, avec le risque de voir se développer des symptômes provenant de l'utilisation de DAV trop longs. En l'absence de critère spécifique, ce risque est pris en aveugle en fonction par exemple de la fraction d'éjection du patient, et en fonction des symptômes éventuellement rapportés lors des visites de suivi, mais en tout état de cause jamais en fonction de la situation instantanée du patient.

D'autre part ces difficultés liées à des DAV longs peuvent n'être que transitoires, et par exemple ne survenir que lors de certains efforts, ou pour certains patients uniquement en période de stimulation atriale et pas après une onde P spontanée. Dans de tels cas, il serait délétère de programmer volontairement des DAV courts de façon définitive pour pallier ces phénomènes transitoires.

Le but de l'invention est de proposer un dispositif pourvu d'un mode de stimulation privilégiant la conduction spontanée du patient (notamment un dispositif du type à hystérésis de DAV ou un dispositif du type AAISafeR ou AAISafeRR), qui pallie l'ensemble des difficultés et limitations exposées ci-dessus, liées à des DAV jugés longs en situation de BAV1.

Le principe de base de l'invention consiste à utiliser un capteur hémodynamique, typiquement (mais de façon non limitative) un capteur d'accélération endocardiaque ou capteur EA, pour surveiller et évaluer la tolérance du patient à des DAV longs, de manière à pouvoir juger si la conduction intrinsèque est bien tolérée ou non, et modifier éventuellement les critères de basculement en mode DDD (ou en mode biventriculaire pour des appareils multisite) pour anticiper au maximum le basculement, ou pour au contraire l'inhiber, en fonction du résultat de cette évaluation. Il s'agit de maximiser les chances de maintenir une conduction ventriculaire spontanée tant qu'elle est bien supportée par le patient, tout en minimisant les risques de DAV longs symptomatiques.

Plus précisément, du point de vue de la mécanique cardiaque le DAV doit être suffisamment long pour permettre à l'oreillette de se contracter complètement et par là même de vider dans le ventricule le sang qu'elle contient, la contraction ventriculaire devant en conséquence se produire après que la contraction atriale ait entièrement eu lieu. Mais elle ne doit pas survenir trop longtemps après, car un DAV trop long risquerait de dissocier le système oreillette/ventricule, avec risque de déclencher des arythmies par conduction rétrograde, ou de réduire l'efficacité hémodynamique du cycle cardiaque. En effet, comme la contraction auriculaire termine le remplissage ventriculaire, le délai entre la fin de ce remplissage et le début de la vidange du ventricule constitue un temps mort, "perdu" sur le plan hémodynamique. De plus, tout délai prolongé de DAV impacte sur la diastole ventriculaire (remplissage du ventricule post-vidange) et donc "recule" d'autant la fin de ce remplissage ventriculaire, qui se superpose alors à la contraction atriale suivante. Il est donc important de pouvoir adapter au mieux le DAV pour chaque patient, de manière que le début de la vidange ventriculaire (provoquée par la stimulation du ventricule) intervienne tout de suite après la fin du remplissage du ventricule par l'oreillette.

Les capteurs hémodynamiques se distinguent des capteurs d'activité (capteurs d'accélération par exemple) et des capteurs métaboliques (capteurs de ventilation-minute par exemple), destinés à diagnostiquer la présence ou non d'un exercice par le patient et à quantifier ses besoins métaboliques, afin par exemple d'adapter la fréquence cardiaque de stimulation en fonction du niveau d'effort détecté.

Les capteurs hémodynamiques (capteur d'accélération endocardiaque, capteur de bioimpédance, etc.) peuvent quant à eux non seulement assurer un suivi des efforts du patient comme les précédents, mais ils peuvent également donner une indication de la tolérance hémodynamique du patient par rapport à certains événements (arythmie atriale ventriculaire, notamment), à des médicaments, ou éventuellement à une modification du DAV.

Le US 5 549 650 A (Bornzin et al./Pacesetter, Inc.) décrit ainsi un stimulateur comprenant un capteur hémodynamique, destiné à adapter une valeur de DAV.

Le US 2006/0293715 décrit une optimisation de la stimulation biventriculaire mettant en oeuvre une analyse de l'accélération endocardiaque.

Mais la technique décrite dans ce document ne vise qu'à modifier la valeur du DAV de manière à optimiser celle-ci en fonction de la réponse du patient. L'objet de la présente invention est au contraire d'utiliser le capteur hémodynamique non pas (ou pas seulement) pour réguler la valeur d'un DAV long, mais pour contrôler la commutation en mode DDD : soit en commutant plus rapidement vers le mode DDD si la valeur du DAV long est - hémodynamiquement parlant - mal tolérée par le patient, soit au contraire pour inhiber la commutation en DDD afin de maintenir la conduction, si le DAV long est bien supporté. De façon similaire, sur un appareil multisite, la présente invention régulera la commutation vers un mode biventriculaire.

Plus précisément, la solution proposée par l'invention consiste à prévoir un dispositif médical implantable actif du type prothèse cardiaque de stimulation, resynchronisation et/ou défibrillation, comprenant de manière en elle-même connue : des moyens de détection d'événements auriculaires et ventriculaires spontanés ; des moyens de stimulation ventriculaire et auriculaire ; des moyens pour faire fonctionner le dispositif en mode DDD ou biventriculaire avec détection ventriculaire et stimulation ventriculaire en l'absence de dépolarisation ventriculaire spontanée détectée à l'issue d'un délai atrio-ventriculaire ; et des moyens de commutation de mode, pour commander conditionnellement, en fonction de critères prédéterminés, le basculement conditionnel du dispositif vers le mode DDD ou biventriculaire depuis un mode de stimulation privilégiant la conduction atrio-ventriculaire spontanée, et inversement le retour vers ce dernier mode depuis le mode DDD ou biventriculaire.

De façon caractéristique de l'invention, le dispositif comprend : un capteur hémodynamique ; des moyens pour dériver du signal délivré par le capteur hémodynamique un indice hémodynamique représentatif de la tolérance du patient à la conduction atrio-ventriculaire spontanée ; et des moyens pour inhiber, ou pour forcer, ledit basculement conditionnel du dispositif vers le mode DDD ou biventriculaire en fonction dudit indice hémodynamique.

Le mode de stimulation privilégiant la conduction atrio-ventriculaire spontanée vers le mode DDD ou biventriculaire peut notamment être un mode AAI avec détection ventriculaire, ou bien un mode DDD ou biventriculaire avec hystérésis du délai atrio-ventriculaire.

Selon diverses mises en oeuvre préférentielles avantageuses :
- le dispositif comprend des moyens de diagnostic aptes à déterminer l'apparition d'un bloc atrio-ventriculaire, et les moyens pour inhiber, ou pour forcer, le basculement conditionnel du dispositif vers le mode DDD ou biventriculaire sont des moyens sélectivement activables, activés seulement en l'absence de bloc atrio-ventriculaire avéré par les moyens de diagnostic ;
- le dispositif comprend des moyens pour évaluer la valeur courante de l'intervalle AR séparant une stimulation auriculaire de la dépolarisation ventriculaire spontanée consécutive, ainsi que des moyens pour comparer la valeur courante de l'intervalle AR à un premier seuil, et les moyens pour inhiber, ou pour forcer, le basculement conditionnel du dispositif vers le mode DDD ou biventriculaire sont des moyens sélectivement activables, activés seulement si la valeur courante de l'intervalle AR est supérieure au premier seuil ;
- dans ce dernier cas, le premier seuil est un seuil variable, fonction de la fréquence cardiaque, et le dispositif comprend des moyens pour réduire dynamiquement la valeur du premier seuil lorsque la fréquence cardiaque courante augmente ;
- le dispositif comprend en outre des moyens de détection d'un état d'effort du patient ; et les moyens pour inhiber, ou pour forcer, le basculement conditionnel du dispositif vers le mode DDD ou biventriculaire sont en outre aptes, après avoir forcé un basculement vers le mode DDD ou biventriculaire, à inhiber le retour au mode de stimulation privilégiant la conduction atrio-ventriculaire spontanée tant que le dispositif détecte un état d'effort du patient ;
- les moyens pour inhiber, ou pour forcer, le basculement conditionnel du dispositif vers le mode DDD ou biventriculaire sont aptes à comparer la valeur courante de l'indice hémodynamique à un indice hémodynamique de référence, et à forcer le basculement du dispositif vers le mode DDD ou biventriculaire lorsque la valeur courante de l'indice hémodynamique est inférieure à l'indice hémodynamique de référence ;
- dans ce dernier cas la valeur de l'indice hémodynamique de référence est une valeur variable en fonction de la fréquence cardiaque, et le dispositif comprend des moyens pour augmenter dynamiquement la valeur de l'indice hémodynamique de référence lorsqu'augmente la fréquence cardiaque courante ;
- cette valeur peut notamment varier entre des limites minimale et maximale, le dispositif comprenant des moyens pour mettre à jour dynamiquement lesdites limites minimale et maximale ;
- le dispositif comprend des moyens de diagnostic aptes à déterminer l'apparition d'un bloc atrio-ventriculaire permanent, et la valeur de l'indice hémodynamique de référence est forcée à une valeur indépendante de la fréquence cardiaque en présence d'un bloc atrio-ventriculaire permanent avéré par les moyens de diagnostic ;
- le capteur d'accélération endocardiaque est un capteur d'accélération endocavitaire ou épicardique, de mouvement d'une paroi du myocarde, de pression intracardiaque, de bioimpédance intracardiaque, de saturation d'oxygène par mesure optique, ou de mesure de variation de volume par ultrasons ;
- dans le cas d'un capteur d'accélération endocardiaque, apte à délivrer un signal représentatif des mouvements produits par les contractions cycliques du myocarde, le dispositif comprend des moyens aptes à reconnaître et isoler dans le signal délivré par le capteur une composante correspondant à un pic d'accélération endocardiaque associé à la contraction ventriculaire, et à dériver ledit indice hémodynamique de l'amplitude de cette composante ;
- dans une forme alternative de cette dernière mise en oeuvre, le dispositif comprend des moyens aptes à reconnaître et isoler dans le signal délivré par le capteur au moins deux composantes correspondant à deux pics respectifs d'accélération endocardiaque associés à la contraction ventriculaire, et à dériver ledit indice hémodynamique d'un intervalle temporel séparant ces deux composantes.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une série de deux chronogrammes illustrant l'électrocardiogramme ECG d'un patient et le signal d'accélération endocardiaque EA correspondant, recueillis au cours d'une série de cycles cardiaques successifs, pour un patient sujet à des BAV1 paroxystiques.
La Figure 2a montre la relation linéaire liant la valeur de référence de l'indice EA à la fréquence cardiaque.
La Figure 2b illustre la relation linéaire liant la valeur seuil de l'intervalle AR au-delà de laquelle le dispositif évalue la tolérance hémodynamique, à la fréquence cardiaque.
La Figure 3 est un organigramme de principe du fonctionnement d'un stimulateur de type AAISafeR, montrant à quel stade intervient le suivi hémodynamique selon l'invention.
La Figure 4 est un organigramme de l'algorithme de suivi de la tolérance hémodynamique selon l'invention.
La Figure 5 est un organigramme de l'algorithme d'apprentissage des valeurs de référence utilisées pour le suivi hémodynamique.
La Figure 6 est une série de deux chronogrammes illustrant l'électrocardiogramme ECG d'un patient et le signal d'accélération endocardiaque EA correspondant, recueillis au cours d'une série de cycles cardiaques successifs, dans le cas d'un patient présentant un BAV1 permanent.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* et *Paradym* produits et commercialisés par ELA Médical, Montrouge, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail. L'invention est applicable à un appareil de stimulation cardiaque ou un défibrillateur implantable équipé d'un algorithme de préservation de la conduction spontanée (par exemple un algorithme du type AAISafeR ou un algorithme à hystérésis de DAV du type DDD-CAM), ainsi que d'un ou plusieurs capteurs physique (G) et/ou physiologique (MV) permettant de différencier les périodes d'activité (effort) et de repos.

De façon caractéristique, le dispositif comporte en outre un capteur hémodynamique permettant d'estimer les variations de contractilité, corrélées aux augmentations de pression sanguine.

Le capteur hémodynamique est avantageusement un capteur d'accélération endocardiaque de type PEA (*Peak Endocardial Acceleration*) tel que celui décrit par exemple dans les EP-A 0 515 319, EP-A 0 582 162 ou EP-A 0 655 260 (tous trois au nom de Sorin Biomedica Cardio SpA) .

Le EP-A-0 515 319 décrit la manière de recueillir un signal d'accélération endocardiaque au moyen d'une sonde endocavitaire pourvue d'une électrode distale de stimulation implantée au fond du ventricule, intégrant en outre un micro-accéléromètre permettant de mesurer l'accélération endocardiaque.

Le EP-A-0 655 260 décrit une manière de traiter le signal d'accélération endocardiaque mesuré pour en dériver notamment deux valeurs de pic d'accélération endocardiaque correspondant aux deux bruits majeurs qu'il est possible de reconnaître dans chaque cycle d'un coeur sain.

Plus précisément, le premier pic d'accélération endocardiaque ("PEA1") correspond à la fermeture des valvules mitrale et tricuspide, au début de la phase de contraction ventriculaire isovolumique (systole). Les variations de ce premier pic sont étroitement liés aux variations de la pression dans le ventricule et peuvent donc constituer un paramètre représentatif de la contractilité du myocarde. L'amplitude du pic PEA1 est, plus précisément, corrélée au maximum positif de la variation de pression dP/dt dans le ventricule gauche.

Le second pic d'accélération endocardiaque ("PEA2"), quant à lui, correspond à la fermeture des valvules aortique et pulmonaire au moment de la phase de relaxation ventriculaire isovolumique. Ce second pic, qui est produit par la décélération brusque de la masse sanguine en mouvement dans l'aorte, constitue un paramètre représentatif de la pression sanguine périphérique au début de la diastole.

En variante, le capteur hémodynamique peut être un capteur d'impédance endocavitaire, par exemple un capteur de mesure de bioimpédance (BioZ) tel que celui divulgué dans les EP-A 1 116 497 ou EP-A 1 138 346 (tous deux au nom d'ELA Medical).

Pus précisément, le EP-A-1 116 497 décrit une manière d'effectuer une mesure dynamique de bioimpédance afin d'évaluer les volumes diastolique et systolique, et obtenir ainsi une indication du débit cardiaque et donc de la fraction d'éjection. Ce document expose en particulier une technique de mesure de la bioimpédance transvalvulaire (entre oreillette et ventricule situés d'un même côté du coeur) par une configuration tripolaire, avec injection d'une impulsion de courant entre un site atrial et un site ventriculaire, et recueil d'un potentiel différentiel entre un site atrial et un site ventriculaire, avec un de ces sites commun à l'injection et au recueil, un site propre d'injection et un site propre de recueil. Le courant injecté est un courant de faible amplitude, insuffisant pour exciter les cellules cardiaques.

Quant au EP-A-1 138 346, il décrit un autre type de mesure de bioimpédance, à savoir une bioimpédance transseptale, c'est-à-dire entre un site situé d'un côté du coeur et un site situé de l'autre côté du coeur. Cette technique permet également de délivrer une valeur représentative de la fraction d'éjection, bien que le signal soit plus faible que dans le cas de la mesure d'une bioimpédance transvalvulaire et soit également influencé par l'impédance propre des tissus du septum.

Dans la description des exemples qui va suivre, on prendra le cas d'un capteur endocavitaire d'accélération endocardiaque, mais ces enseignements sont transposables aux signaux délivrés par d'autres types de capteur d'accélération, par exemple un capteur épicardique ou un capteur de mouvement d'une paroi du myocarde, ou de façon générale par tout autre type de capteur capable de délivrer un signal représentatif du comportement hémodynamique du myocarde, par exemple un capteur de bioimpédance intracardiaque ou un capteur de pression.

Ce capteur est destiné à évaluer la tolérance hémodynamique du patient aux DAV longs afin non seulement de moduler la valeur du DAV, mais surtout de commuter plus rapidement en mode DDD si la valeur est mal tolérée ou, à l'inverse, de préserver la conduction intrinsèque et empêcher ou retarder le basculement en mode DDD si le DAV long est supporté, hémodynamiquement parlant.

Un DAV long correspond à une situation potentielle de BAV1. Plus précisément, un BAV du premier degré ou BAV1 correspond à une conduction présente, mais retardée. Il se distingue : (i) du BAV du deuxième degré (BAV2), qui se caractérise par une conduction incomplète avec un allongement progressif de l'intervalle PR (ou AR) tel qu'une partie des ondes P ne sont plus conduites ; (ii) du BAV complet ou BAV du troisième degré (BAV3) qui se manifeste par des ondes auriculaires (stimulées ou spontanées) totalement bloquées, c'est-à-dire qui ne sont plus suivies de dépolarisations ventriculaires ; et (iii) de la pause ventriculaire, lorsque l'intervalle séparant deux événements ventriculaires excède un délai donné, par exemple excède trois secondes, que ce phénomène trouve ou non son origine dans un trouble de la conduction auriculo-ventriculaire.

Un BAV1 peut être paroxystique ou permanent. Un BAV1 paroxystique présente un caractère intermittent, et peut survenir typiquement lors de phases d'effort ou de sommeil, et disparaître spontanément en fin d'effort ou à l'éveil. En revanche, un BAV permanent ou quasi-permanent est révélateur d'un trouble chronique devant être pris en compte de manière adaptée.

### Mise en oeuvre de l'invention en cas de BAV1 paroxystiques

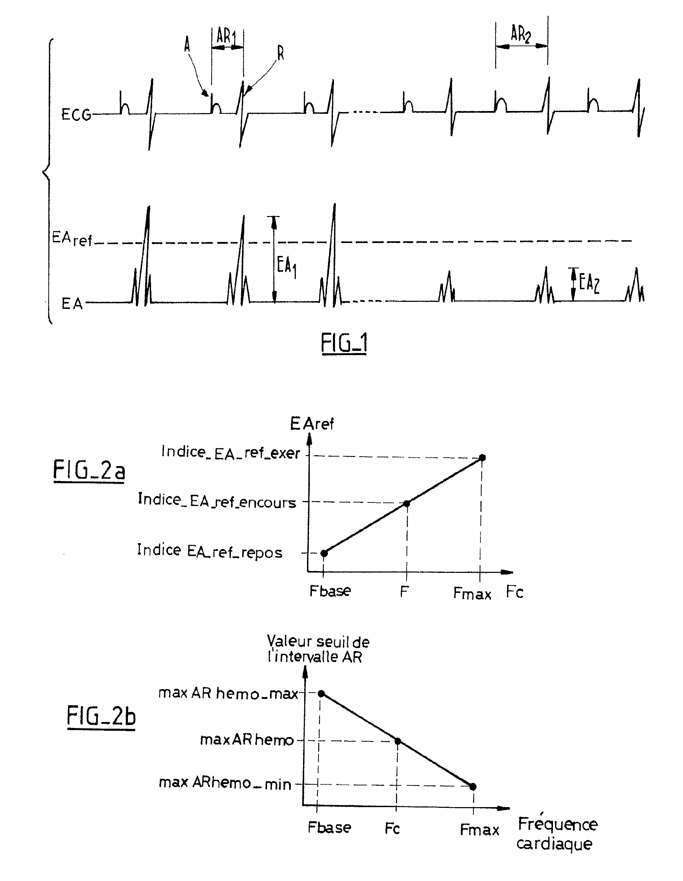

Sur la Figure 1, le chronogramme supérieur présente un électrocardiogramme (ECG) endocavitaire avec, pour chaque cycle cardiaque, une onde auriculaire stimulée A, suivie d'une onde de dépolarisation ventriculaire spontanée R. Dans les premiers cycles, le délai *AR₁* entre stimulation A et détection R est plus court que le délai correspondant *AR₂* dans les derniers cycles, révélant un allongement du délai de conduction auriculo-ventriculaire intrinsèque.

Le chronogramme inférieur présente un signal hémodynamique correspondant, typiquement le signal d'accélération endocardiaque (EA). Dans l'exemple illustré ici, on choisit comme paramètre représentatif l'amplitude *EA₁, EA₂* ... du signal EA à l'instant du complexe QRS, c'est-à-dire le premier pic d'accélération endocardiaque PEA1 correspondant au premier bruit majeur, au début de la phase de contraction ventriculaire isovolumique. On sait en effet que les variations d'amplitude de ce premier pic sont étroitement liées aux variations de la pression dans le ventricule et peuvent donc constituer un paramètre représentatif de la contractilité du myocarde.

La ligne en tiretés indique une amplitude de référence *EA_{ref}* permettant de différencier une bonne tolérance hémodynamique (*EA₁ > EA_{ref}*) et une mauvaise tolérance hémodynamique (*EA₂* < *EA_{ref}*). On voit ainsi que, pour le délai AR₁ l'amplitude *EA₁* est supérieure à la valeur de référence indiquant donc que ce délai AR₁ est bien toléré et ne nécessite aucune intervention particulière du dispositif. En revanche, pour le délai AR₂ plus long que AR₁, l'amplitude *EA₂* correspondante est inférieure à la valeur de référence *EA_{ref}* : ceci indique que le délai AR₂ est mal toléré, et nécessite le recours à une stimulation ventriculaire pour éviter l'apparition de symptômes liés à un BAV1.

Le principe de l'invention consiste à utiliser cette valeur d'amplitude du pic du signal EA (et/ou d'autres paramètres pertinents tels qu'amplitude du deuxième pic, intervalle entre premier et deuxième pic, etc.) en tant qu'indice ou marqueur de la tolérance hémodynamique du patient à des DAV longs.

Ce marqueur sera désigné *Indice_EA*. Afin de définir une valeur de référence, le dispositif enregistre au préalable des moyennes de cet indice au repos et à l'effort, et ce pour des valeurs de DAV normales, c'est-à-dire en l'absence de situation de BAV1 (donc pour des valeurs de DAV inférieures à une valeur seuil, ci-après désignée *MaxARhemo* au-delà de laquelle on évaluera la tolérance hémodynamique, comme cela sera exposé plus en détail par la suite). La valeur d'indice référence ainsi déterminée sera une valeur variable en fonction de l'activité du patient, par exemple une variation linéaire entre une valeur de repos et une valeur d'effort. Une fois cette valeur de référence définie, et lorsque l'on se trouve en situation de DAV long (délai AR supérieur au seuil *MaxARhemo*), le dispositif compare l'indice courant à l'indice de référence :
- si l'indice courant est inférieur à l'indice de référence, le dispositif considère qu'il y a absence de tolérance hémodynamique, que l'on se trouve en situation de BAV1 symptomatique, et qu'il est nécessaire de stimuler le ventricule pour retrouver une situation hémodynamique satisfaisante. Le dispositif opère alors une commutation en mode DDD (dans le cas d'un dispositif de type AAISafeR) ou en mode biventriculaire (dans le cas d'un appareil multisite), ou raccourcit le DAV (dans le cas d'un dispositif à hystérésis du DAV).
   Dans le reste de la description par "commutation en mode DDD", on entendra également une commutation en mode biventriculaire dans le cas d'un appareil multisite.
   Le dispositif reste dans cet état pendant une durée prédéterminée et/ou jusqu'à la fin de la situation d'effort, puis recommute en mode AAI (ou rallonge à nouveau le DAV dans le cas d'un dispositif à hystérésis).
- dans le cas contraire, le dispositif considère que la conduction intrinsèque est bien tolérée par le patient, et force le dispositif à rester en mode AAI (ou inhibe le raccourcissement du délai dans le cas d'un dispositif à hystérésis).

On va maintenant décrire plus en détail ce mode opératoire, en référence aux Figures 2 à 5.

La Figure 2a explicite la relation liant la valeur de référence courante de *EA_{ref}*, notée *Indice_EA_ref_encours*, à la fréquence cardiaque *F_{c}.* En effet, si pour certains choix d'indices hémodynamiques la valeur de référence peut être fixe, dans le cas où l'on choisit pour cet indice l'amplitude du premier pic du signal EA, il est souhaitable de modifier la valeur du seuil *EA_{ref}* en fonction du niveau de l'effort. En effet, comme l'amplitude du premier pic du signal EA augmente lorsque la fréquence cardiaque augmente, il est logique d'adapter en conséquence la valeur du seuil *EA_{ref}.* Comme on peut le voir sur la Figure 2a, cet indice *EA_{ref}* varie linéairement entre une valeur minimale (désignée *Indice_EA_ref_repos*), correspondant à une fréquence cardiaque proche de la fréquence de base *F_{base},* et une valeur maximale (désignée *Indice_EA_ref_exer*) utilisée lorsque la fréquence cardiaque est proche de la fréquence maximale *Fₘₐₓ.* Entre ces deux extrêmes, à chaque valeur instantanée F de la fréquence cardiaque *F_{c}* correspond une valeur instantanée de l'indice de référence *EA_{ref},* désignée *Indice_EA_ref_encours*. On notera que la relation linéaire a été choisie pour sa simplicité, mais cette relation n'est pas limitative, et à d'autres indices ou autres types de capteurs hémodynamiques peuvent correspondre d'autres relations, non linéaires.

La Figure 2b, quant à elle, explicite la relation liant la valeur seuil de l'intervalle AR au-delà de laquelle on analysera la tolérance hémodynamique, à la fréquence cardiaque instantanée *F_{c}.*

Cette valeur seuil, désignée *MaxARhemo,* est le délai AR limite au-delà duquel le dispositif estimera que le patient est en risque de BAV1, et donc au-delà duquel il conviendra d'analyser la tolérance hémodynamique à ce délai AR long.

Dans certaines mises en oeuvre simplifiées de l'invention, cette valeur peut être fixe, par exemple de l'ordre de 400 ou 450 ms. Toutefois, même si cette valeur fixe est bien adaptée à une situation de repos, elle peut être insuffisante lors d'un effort : en effet, l'intervalle PR physiologique se raccourcit lorsque la fréquence cardiaque augmente, de sorte qu'un délai de 300 ms peut être tout à fait acceptable dans une situation de repos, mais beaucoup trop long dans une situation d'effort.

Comme illustré sur la Figure 2b, le dispositif fait donc avantageusement varier le délai limite *MaxARhemo* entre une valeur maximale (désignée *MaxARhemo_max*) appliquée lorsque la fréquence cardiaque *F_{c}* est proche de la fréquence de base *F_{base},* et une valeur minimale (désignée *MaxARhemo_min*) appliquée lorsque la fréquence cardiaque *F_{c}* est proche de la fréquence maximale *Fₘₐₓ*, en suivant de préférence une variation linéaire. Entre les deux, à chaque fréquence cardiaque instantanée *F_{c}* correspondra une valeur instantanée *MaxARhemo* du délai limite au-delà de laquelle on évaluera la tolérance hémodynamique du BAV1.

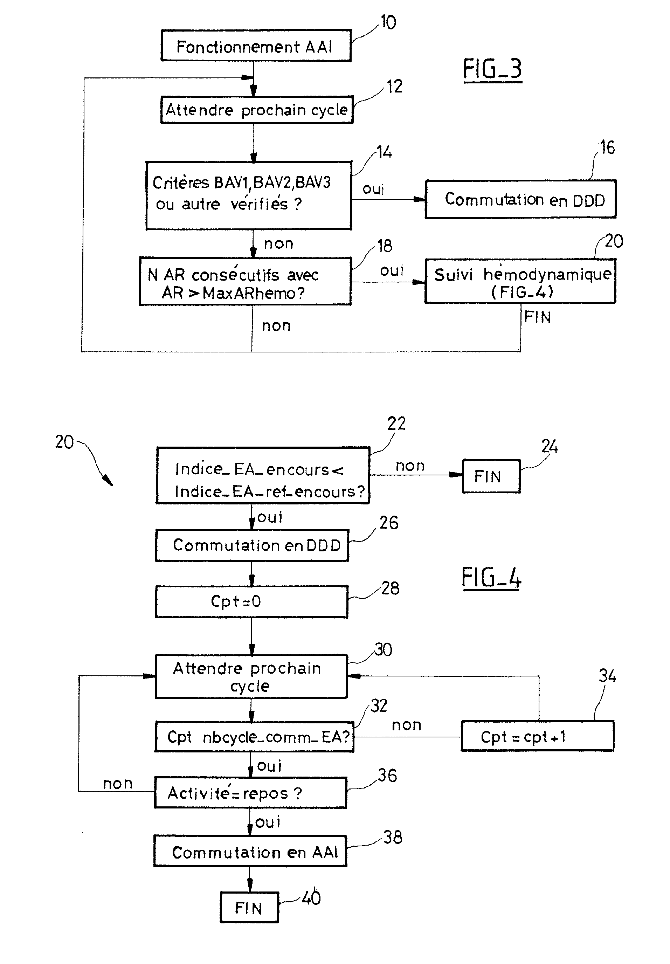

La Figure 3 est un organigramme illustrant la manière dont l'invention peut être mise en oeuvre au sein d'un dispositif comprenant un mode de fonctionnement du type AAISafeR indiqué plus haut (ce principe pouvant être appliqué *mutatis mutandis* aux dispositifs utilisant un algorithme d'adaptation de l'hystérésis du DAV).

L'invention est mise en oeuvre lorsque le stimulateur est en situation de fonctionnement AAI (bloc 10). L'appareil attend (bloc 12) la fin du cycle ventriculaire en cours pour analyser l'état de la conduction. Il évalue ensuite (bloc 14) si les critères de suspicion de BAV1, BAV2 ou BAV3 (ou autre critère) sont vérifiés. Ces critères sont par exemple :
a) BAV du premier degré ou BAV1 (conduction présente, mais retardée) : le nombre d'évènements auriculaires suivis d'une détection ventriculaire survenant, par exemple, après un délai supérieur à 350 ms (pour un évènement auriculaire spontané) ou 450 ms (pour un évènement auriculaire stimulé) dépasse un nombre donné, par exemple six cycles cardiaques consécutifs ;
b) BAV du deuxième degré ou BAV2 (conduction incomplète, l'allongement progressif de l'intervalle PR, ou AR, étant tel qu'une partie des ondes P ne sont plus conduites) : le nombre d'évènements auriculaires non suivis d'une détection ventriculaire excède un certain nombre sur la durée d'une fenêtre de surveillance s'étendant sur un nombre prédéterminé d'évènements auriculaires : par exemple lorsque le dispositif détecte trois ondes P bloquées non consécutives parmi les douze derniers cycles cardiaques ;
c) BAV complet, du troisième degré, ou BAV3 (ondes auriculaires, stimulées ou spontanées, totalement bloquées, c'est-à-dire qui ne sont plus suivies de dépolarisations ventriculaires) : succession de deux ondes auriculaires, détectées ou stimulées, bloquées, ou écoulement de plus de trois secondes sans détection ventriculaire (situation de pause ventriculaire).

Si l'un de ces critères (ou autre critère) est vérifié, alors l'algorithme commute l'appareil en mode DDD (bloc 16), de façon en elle-même conforme à un fonctionnement AAISafeR classique.

En revanche, si aucun des critères habituels de détection des BAV1, BAV2 ou BAV3 n'est vérifié, l'algorithme évalue les durées des *N* derniers délais AR (bloc 18). Selon les mises en oeuvre particulières, cette évaluation peut correspondre au calcul d'une valeur moyenne, à la recherche d'une valeur maximale au sein des *N* valeurs ou, comme dans le présent exemple, à un critère basé sur le dépassement par *N* délais AR consécutifs de la valeur instantanée du seuil *MaxARhemo,* ce seuil *MaxARhemo* étant choisi de manière à être toujours inférieur au seuil du critère traditionnel de BAV1 (350 ou 450 ms).

Si ce critère est vérifié, ceci signifie que le patient n'est pas en situation de BAV1 au regard des critères traditionnels mais est en situation de BAV1 au regard du critère selon l'invention. L'algorithme va alors, de façon caractéristique de l'invention, évaluer la tolérance du patient à ce BAV1 en déclenchant un suivi de la tolérance hémodynamique (bloc 20, qui sera exposée en détail à la Figure 4).

C'est dans cette phase de suivi de la tolérance hémodynamique que l'algorithme forcera le dispositif à rester en mode AAI, ou au contraire imposera un basculement en mode DDD, en fonction du résultat de l'analyse de la tolérance hémodynamique.

Si, en revanche, le critère évalué au bloc 18 n'est pas vérifié, cela signifie que le patient n'est pas en situation de BAV1. Aucune action spécifique n'est prise, et l'algorithme retourne au bloc 12 pour analyser le prochain cycle cardiaque.

La Figure 4 est un organigramme décrivant en détail la manière dont est opéré le suivi de la tolérance hémodynamique correspondant au bloc 20 de la Figure 3, c'est-à-dire en situation de BAV1 et en présence de délais AR supérieurs à la valeur seuil *MaxARhemo.*

Tout d'abord (bloc 22), l'algorithme compare la valeur courante de l'indice *Indice_EA,* désignée *Indice_EA_encours*, mesurée à partir du capteur hémodynamique, à la valeur instantanée de référence, à savoir la valeur *Indice_EA_ref_encours.* S'il est supérieur ou égal à la référence, ceci signifie que le BAV1 est bien toléré. Il n'y a pas lieu de commuter en DDD et l'algorithme peut retourner (bloc 24) au bloc 12 de la Figure 3.

Dans le cas contraire, l'algorithme commande une commutation en mode DDD (bloc 26), avec hystérésis éventuelle pour préserver une marge suffisante correspondant par exemple à la variation physiologique des signaux.

Au bloc 28, un compteur est remis à zéro, qui permettra de maintenir le mode DDD pendant un nombre prédéfini de cycles qui peut être programmable, par exemple 100 cycles cardiaques.

Au bloc 30, l'algorithme attend le prochain complexe ventriculaire pour remettre à jour les données correspondant au temps passé en mode DDD. Au bloc 32, le compteur est comparé à la valeur prédéfinie évoquée plus haut (les 100 cycles cardiaques par exemple). Si cette valeur n'est pas atteinte, le dispositif reste en mode DDD, le compteur est augmenté d'une unité (bloc 34) et l'algorithme retourne au bloc 30.

Dans le cas contraire, l'algorithme teste (bloc 36) si le patient est en phase de repos ou d'exercice au moyen des capteurs d'activité du dispositif.

Si le patient n'est pas au repos, alors le dispositif est maintenu en mode DDD jusqu'à détection d'une phase de repos du patient (retour au bloc 30). En effet, chez des patients en dysfonction sinusale, l'effort est l'un des principaux facteurs déclenchants de BAV1, de sorte qu'il est judicieux de maintenir le mode DDD tout au long d'un effort, afin d'optimiser la tolérance pendant toute la durée de cette phase.

Dans le cas contraire, c'est-à-dire lorsqu'un repos est diagnostiqué au bloc 36, alors l'algorithme recommute le dispositif en mode AAI (bloc 38) et retourne (bloc 40) au bloc 12 de la Figure 3 en attente du prochain cycle.

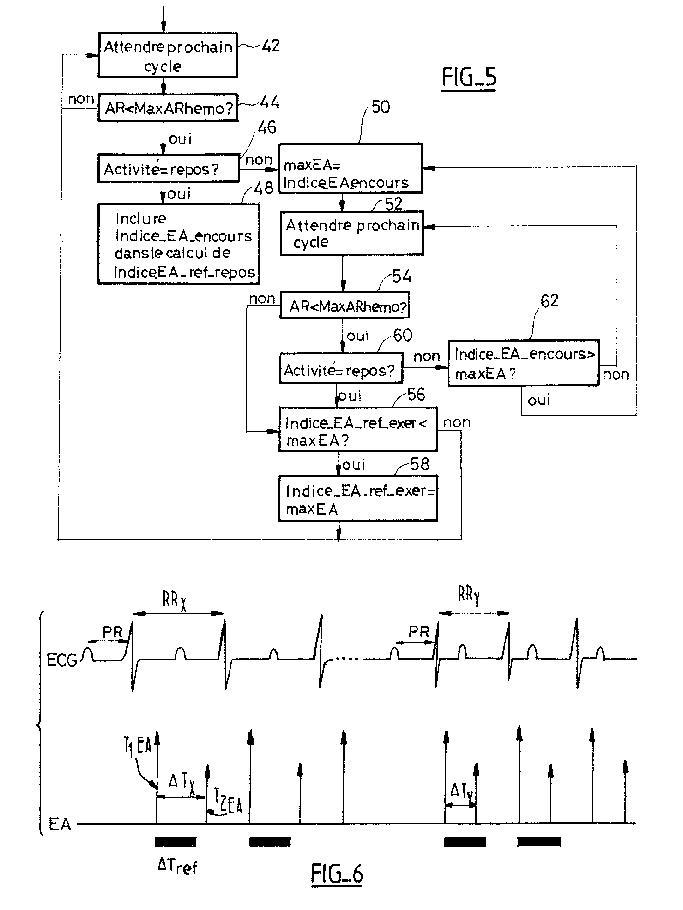

La Figure 5 illustre la manière dont sont mises à jour les valeurs de référence du capteur hémodynamique.

Cette mise à jour se déroule parallèlement aux procédures décrites plus haut, et elle est exécutée lorsque le dispositif est en mode AAI, en l'absence de détection d'un BAV1.

Comme on l'a expliqué plus haut en référence à la Figure 2a, l'indice de référence *EA_{ref}* est avantageusement modulable entre une valeur minimale *Indice_EA_ref_repos* et une valeur maximale *Indice_EA_ref_exer*, la valeur courante *Indice_EA_ref_encours* variant de façon permanente et dynamique entre ces deux extrêmes.

Au départ, l'algorithme attend la fin du cycle ventriculaire en cours pour procéder à l'analyse (bloc 42).

Il compare alors (bloc 44) le délai AR courant au seuil *MaxARhemo* courant. Si le délai AR est supérieur à *MaxARhemo,* ceci signifie qu'il y a situation de BAV1, et les données de référence ne sont pas remises à jour (comme indiqué plus haut, cette mise à jour n'est opérée qu'en l'absence de détection de BAV1). L'algorithme retourne donc au bloc 42.

Dans le cas contraire, la procédure de mise à jour peut débuter.

L'algorithme teste tout d'abord (bloc 46) si le patient est au repos ou à l'exercice.

Si le patient est au repos, l'algorithme inclut dans le calcul général de *In-dice_EA_ref repos* la valeur courante de *Indice_EA_encours* (bloc 48). Ce calcul peut être par exemple une moyenne journalière, mais il est également possible de retenir pour *Indice_EA_ref_repos* la valeur minimale ou la valeur maximale constatée au repos sur une période donnée.

Si le patient est en activité, l'algorithme procède alors à la mise à jour de la valeur *Indice_EA_ref_exer*.

A cet effet, une valeur *maxEA* est initialisée avec la valeur courante de *Indice*_*EA*_*encours* (bloc 50). L'algorithme se met ensuite en attente du prochain cycle ventriculaire (bloc 52) puis compare à nouveau (bloc 54) la valeur du délai AR à la valeur courante de *MaxARhemo,* de la même manière qu'au bloc 44. Si le délai AR est supérieur à *MaxARhemo,* ceci signifie que l'on est en situation de BAV1, et la mise à jour des données de référence est interrompue. Avant d'achever la mise à jour, l'algorithme compare *Indice_EA_ref_exer à maxEA* (bloc 56). Si cette dernière valeur est supérieure, l'algorithme met à jour *Indice_EA_ref_exer* avec cette nouvelle valeur *maxEA* (bloc 58). Sinon, la mise à jour est interrompue et l'algorithme retourne directement au bloc 42 en attente du cycle suivant.

Si, au bloc 54, la valeur du délai AR est inférieure à la valeur courante de *MaxARhemo,* ceci signifie qu'il n'y a pas situation de BAV1, et la mise à jour peut continuer.

L'algorithme vérifie si le patient est retourné au repos (bloc 60). Si tel est le cas, il met fin à la mise à jour de la valeur d'effort, et les valeurs de référence sont mises à jour dans les blocs 56 et 58 comme décrit plus haut en situation de BAV1. Dans le cas contraire (patient toujours en exercice), l'algorithme compare (bloc 62) la valeur de *Indice_EA_ref_encours* à la valeur de *maxEA.* Si cette dernière valeur est inférieure, alors la valeur de *maxEA* est mise à jour avec la nouvelle valeur *Indice_EA_ref_encours* (bloc 50), sinon aucune mise à jour de *maxEA* n'est nécessaire et l'algorithme se met en attente (bloc 52) du cycle ventriculaire suivant.

On notera que la procédure que l'on vient de décrire permet d'obtenir pour l'indice de référence EA_{ref} une valeur moyennée au repos pour la limite inférieure *Indice_EA_ref_repos*, et la valeur maximale à l'effort pour la limite supérieure *Indice_EA_ref_exer.* Inévitablement, un tel système dévierait au fil des itérations, de sorte que pour éviter cette dérive le paramètre *maxEA* est abaissé d'un incrément à intervalles réguliers (par exemple toutes les 24 heures) de manière à rééquilibrer le système;

### Mise en oeuvre de l'invention en cas de BAV1 permanent

Le mode opératoire que l'on vient de décrire n'est efficace que si le patient souffre de BAV1 paroxystiques, notamment en ce qui concerne l'apprentissage des valeurs de référence. En effet, en cas de BAV1 permanent, il est impossible de mettre à jour l'indice EA_{ref}.

Il est donc nécessaire de choisir un autre indice de référence dérivé du signal EA, par exemple un temps de remplissage. L'indice de référence sera dans ce cas une simple valeur seuil, qui ne variera pas nécessairement avec la fréquence.

Cette situation est illustrée Figure 6.

En cas de BAV1 permanent, les délais AR restent toujours longs, seules les autres conditions varient, par exemple la fréquence cardiaque qui augmente, ce qui se traduit par un raccourcissement du délai RR (*RRₓ < RR_{y}* sur la Figure 6).

Le paramètre utilisé dérivé du signal EA peut être par exemple la durée Δ*Tₓ* séparant les deux pics PEA1 et PEA2 du signal EA au cours d'un même cycle cardiaque, ces pics étant repérés par les marqueurs temporels T_{1EA} et T_{2EA} sur la Figure 6. L'intervalle Δ*Tₓ* sera comparé à un seuil de référence, fixe, Δ*T_{ref}*, et l'on considérera que le BAV1 est bien supporté si Δ*Tₓ* > Δ*T_{ref}*, et mal supporté sinon. Dans ce dernier cas, l'algorithme commutera le dispositif en mode DDD, car la situation du BAV1 impose le passage à ce mode de stimulation. Le reste du fonctionnement, pour le retour en AAI, peut être identique au cas précédent.

Les marqueurs temporels T_{1EA} et T_{2EA} peuvent être notamment déterminés par mise en oeuvre de la technique décrite dans la demande européenne n° 09 209116.1 du 18.02.2009, déposée sous priorité de la demande française 08 00907 du 20.02.2008, pour "*Dispositif d'analyse d'un signal d'accélération endocardiaque*", au nom de ELA Medical, qui explique la manière de déterminer la position temporelle des diverses composantes associées aux bruits cardiaques S1, S2, S3 ou S4 d'un signal EA d'accélération endocardiaque et notamment, mais de façon non limitative, aux composantes EA1 et EA2 ou également aux "pics" d'accélération endocardiaque PEA1 et PEA2. L'intervalle Δ*T* est calculé en permanence, soit en valeur absolue soit par ses variations relatives à une composante mécanique cardiaque telle que le temps de remplissage.

Sur la Figure 6, on voit que le patient est en situation de BAV1, avec un délai PR constant caractéristique. En revanche, au fur et à mesure que la fréquence cardiaque augmente, le délai RR diminue (*RR_{y}* < *RRₓ*), avec des délais hémodynamiques également en diminution (Δ*T_{y}*< Δ*Tₓ*). La valeur Δ*T_{ref}* choisie comme seuil de tolérance peut être notamment une valeur correspondant au temps de remplissage minimum assurant pour le patient une hémodynamique cardiaque correcte.

Sur la Figure 6, initialement Δ*Tₓ* > Δ*T_{ref}*, ce qui signifie que le BAV1 est bien toléré, et le dispositif peut donc rester en mode de fonctionnement AAI. En revanche, ultérieurement Δ*T_{y}* < Δ*T_{ref}*, ce qui signifie que le temps de remplissage est insuffisant. Le BAV1 est donc mal supporté par le patient, et le dispositif doit opérer un repli en mode DDD (le retour ultérieur en mode AAI pouvant s'effectuer selon les mêmes procédures que celles décrites dans le cadre de la Figure 4).

## Revendications

1. Un dispositif médical implantable actif du type prothèse cardiaque de stimulation, resynchronisation et/ou défibrillation, comprenant :
- des moyens de détection d'événements auriculaires et ventriculaires spontanés ;
- des moyens de stimulation ventriculaire et auriculaire ;
- des moyens pour faire fonctionner le dispositif en mode DDD ou biventriculaire avec détection ventriculaire et stimulation ventriculaire en l'absence de dépolarisation ventriculaire spontanée détectée à l'issue d'un délai atrio-ventriculaire (DAV) ; et
- des moyens de commutation de mode, pour commander conditionnellement, en fonction de critères prédéterminés, le basculement conditionnel du dispositif vers le mode DDD ou biventriculaire depuis un mode de stimulation privilégiant la conduction atrio-ventriculaire spontanée, et inversement le retour vers ce dernier mode depuis le mode DDD ou biventriculaire,
dispositif **caractérisé en ce qu'**il comprend :
- un capteur hémodynamique ;
- des moyens pour dériver du signal délivré par le capteur hémodynamique un indice hémodynamique (*Indice_EA_encours*) représentatif de la tolérance du patient à la conduction atrio-ventriculaire spontanée ; et
- des moyens (20) pour inhiber, ou pour forcer, ledit basculement conditionnel du dispositif vers le mode DDD ou biventriculaire en fonction dudit indice hémodynamique.

2. Le dispositif de la revendication 1, dans lequel le mode de stimulation privilégiant la conduction atrio-ventriculaire spontanée vers le mode DDD ou biventriculaire est un mode AAI avec détection ventriculaire.

3. Le dispositif de la revendication 1, dans lequel le mode de stimulation privilégiant la conduction atrio-ventriculaire spontanée vers le mode DDD ou biventriculaire est un mode DDD ou biventriculaire avec hystérésis du délai atrio-ventriculaire.

4. Le dispositif de la revendication 1, dans lequel :
- le dispositif comprend des moyens de diagnostic (14) aptes à déterminer l'apparition d'un bloc atrio-ventriculaire, et
- les moyens (20) pour inhiber, ou pour forcer, le basculement conditionnel du dispositif vers le mode DDD ou biventriculaire sont des moyens sélectivement activables, activés seulement en l'absence de bloc atrio-ventriculaire avéré par les moyens de diagnostic.

5. Le dispositif de la revendication 1, dans lequel :
- le dispositif comprend des moyens pour évaluer la valeur courante de l'intervalle AR (*AR₁, AR₂*) séparant une stimulation auriculaire (A) de la dépolarisation ventriculaire spontanée (R) consécutive ;
- le dispositif comprend des moyens (18) pour comparer la valeur courante de l'intervalle AR à un premier seuil (*MaxARhemo*), et
- les moyens (20) pour inhiber, ou pour forcer, le basculement conditionnel du dispositif vers le mode DDD ou biventriculaire sont des moyens sélectivement activables, activés seulement si la valeur courante de l'intervalle AR est supérieure au premier seuil.

6. Le dispositif de la revendication 5, dans lequel :
- le premier seuil (*MaxARhemo*) est un seuil variable, fonction de la fréquence cardiaque (*F_{c}*), et
- le dispositif comprend des moyens pour réduire dynamiquement la valeur du premier seuil lorsque la fréquence cardiaque courante augmente.

7. Le dispositif de la revendication 1, dans lequel :
- le dispositif comprend en outre des moyens (36) de détection d'un état d'effort du patient ; et
- les moyens (20) pour inhiber, ou pour forcer, le basculement conditionnel du dispositif vers le mode DDD ou biventriculaire sont en outre aptes, après avoir forcé (26) un basculement vers le mode DDD ou biventriculaire, à inhiber le retour (38) au mode de stimulation privilégiant la conduction atrio-ventriculaire spontanée tant que le dispositif détecte un état d'effort du patient.

8. Le dispositif de la revendication 1, dans lequel les moyens (20) pour inhiber, ou pour forcer, le basculement conditionnel du dispositif vers le mode DDD ou biventriculaire :
- sont aptes à comparer (22) la valeur courante de l'indice hémodynamique (*Indice_EA_encours*) à un indice hémodynamique de référence (*Indice_EA_ref_encours*) ; et
- sont aptes à forcer (26) le basculement du dispositif vers le mode DDD ou biventriculaire lorsque la valeur courante de l'indice hémodynamique est inférieure à l'indice hémodynamique de référence.

9. Le dispositif de la revendication 8, dans lequel :
- la valeur de l'indice hémodynamique de référence (*EA_{ref}*) est une valeur variable en fonction de la fréquence cardiaque (*F_{c}*), et
- le dispositif comprend des moyens pour augmenter dynamiquement la valeur de l'indice hémodynamique de référence lorsqu'augmente la fréquence cardiaque courante.

10. Le dispositif de la revendication 9, dans lequel :
- la valeur de l'indice hémodynamique de référence (*EA_{ref}*) est une valeur variable entre des limites minimale (*Indice_EA_ref_repos*) et maximale (*Indice_EA_ref_exer*), et
- le dispositif comprend des moyens (42-62) pour mettre à jour dynamiquement lesdites limites minimale et maximale.

11. Le dispositif de la revendication 8, dans lequel :
- le dispositif comprend des moyens de diagnostic aptes à déterminer l'apparition d'un bloc atrio-ventriculaire permanent, et
- la valeur de l'indice hémodynamique de référence (*EA_{ref}*) est forcée à une valeur indépendante de la fréquence cardiaque en présence d'un bloc atrio-ventriculaire permanent avéré par les moyens de diagnostic.

12. Le dispositif de la revendication 1, dans lequel le capteur d'accélération endocardiaque est un capteur du groupe formé par : capteur d'accélération endocavitaire ou épicardique ; capteur de mouvement d'une paroi du myocarde ; capteur de pression intracardiaque ; capteur de bioimpédance intracardiaque ; capteur de saturation d'oxygène par mesure optique ; capteur de mesure de variation de volume par ultrasons.

13. Le dispositif de la revendication 12, dans lequel le capteur hémodynamique est un capteur d'accélération endocardiaque, apte à délivrer un signal (EA) représentatif des mouvements produits par les contractions cycliques du myocarde.

14. Le dispositif de la revendication 13, dans lequel le dispositif comprend des moyens aptes à reconnaître et isoler dans le signal délivré par le capteur une composante (EA₁) correspondant à un pic d'accélération endocardiaque associé à la contraction ventriculaire, et à dériver ledit indice hémodynamique de l'amplitude de cette composante.

15. Le dispositif de la revendication 13, dans lequel le dispositif comprend des moyens aptes à reconnaître et isoler dans le signal délivré par le capteur au moins deux composantes (T_{1EA}, T_{2EA}) correspondant à deux pics respectifs d'accélération endocardiaque associés à la contraction ventriculaire, et à dériver ledit indice hémodynamique d'un intervalle temporel (Δ*T*_{*x*,}) séparant ces deux composantes.

## Patentansprüche

1. Aktive implantierbare medizinische Vorrichtung vom Typ Herzprothese zur Stimulation, Resynchronisation und/oder Defibrillation, umfassend:
- Mittel zum Detektieren von spontanen atrialen und ventrikulären Ereignissen;
- Mittel zur ventrikulären und atrialen Stimulation; und
- Mittel, um die Vorrichtung im biventrikulären oder DDD-Modus mit ventrikulärer Detektion und ventrikulärer Stimulation in Abwesenheit von spontaner ventrikulärer Depolarisation, die am Ende einer atrioventrikulären Verzögerung (DAV) detektiert wird, arbeiten zu lassen; und
- Mittel zum Wechseln des Modus, um in Abhängigkeit von vorbestimmten Kriterien das bedingte Umschalten der Vorrichtung in den biventrikulären oder DDD-Modus von einem Stimulationsmodus, der die spontane atrioventrikuläre Leitung bevorzugt, und umgekehrt die Rückkehr zu letztgenanntem Modus vom biventrikulären oder DDD-Modus, bedingt zu steuern,
wobei die Vorrichtung **dadurch gekennzeichnet ist, dass** sie umfasst:
- einen hämodynamischen Sensor;
- Mittel, um von dem Signal, das von dem hämodynamischen Sensor gesendet wird, eine hämodynamische Kennzahl (*Indice*_*EA*_*encours*) abzuleiten, die für die Toleranz des Patienten bezüglich der spontanen atrioventrikulären Leitung repräsentativ ist; und
- Mittel (20), um das bedingte Umschalten der Vorrichtung in den biventrikulären oder DDD-Modus in Abhängigkeit von der hämodynamischen Kennzahl zu inhibieren oder zu erzwingen.

2. Vorrichtung nach Anspruch 1, wobei der Stimulationsmodus, der die spontane atrioventrikuläre Leitung in den biventrikulären oder DDD-Modus bevorzugt, ein AAI-Modus mit ventrikulärer Detektion ist.

3. Vorrichtung nach Anspruch 1, wobei der Stimulationsmodus, der die spontane atrioventrikuläre Leitung in den biventrikulären oder DDD-Modus bevorzugt, ein biventrikulärer oder DDD-Modus mit Hysterese der atrioventrikulären Verzögerung ist.

4. Vorrichtung nach Anspruch 1, wobei:
- die Vorrichtung Diagnosemittel (14) umfasst, die geeignet sind, um das Auftreten eines atrioventrikulären Blocks zu bestimmen, und
- die Mittel (20), um das bedingte Umschalten der Vorrichtung in den biventrikulären oder DDD-Modus zu inhibieren oder zu erzwingen, selektiv aktivierbare Mittel sind, die nur in Abwesenheit eines atrioventrikulären Blocks, der durch die Diagnosemittel ermittelt wird, aktiviert werden.

5. Vorrichtung nach Anspruch 1, wobei:
- die Vorrichtung Mittel zum Evaluieren des aktuellen Werts des Intervalls AR (*AR₁*, *AR₂*) umfasst, das eine atriale Stimulation (A) von der folgenden spontanen ventrikulären Depolarisation (R) trennt;
- die Vorrichtung Mittel (18) umfasst, um den aktuellen Wert des Intervalls AR mit einem ersten Schwellenwert (*MaxARhemo*) zu vergleichen, und
- die Mittel (20), um das bedingte Umschalten der Vorrichtung in den biventrikulären oder DDD-Modus zu inhibieren oder zu erzwingen, Mittel sind, die selektiv aktivierbar sind, die nur aktiviert werden, wenn der aktuelle Wert des Intervalls AR größer ist als der erste Schwellenwert.

6. Vorrichtung nach Anspruch 5, wobei:
- der erste Schwellenwert (*MaxARhemo*) ein variabler Schwellenwert ist, der von der Herzfrequenz (*F_{c}*) abhängig ist, und
- die Vorrichtung Mittel zum dynamischen Reduzieren des Werts des ersten Schwellenwerts umfasst, wenn sich die aktuelle Herzfrequenz erhöht.

7. Vorrichtung nach Anspruch 1, wobei:
- die Vorrichtung ferner Mittel (36) zum Detektieren eines Anstrengungszustands des Patienten umfasst; und
- die Mittel (20), um das bedingte Umschalten der Vorrichtung in den biventrikulären oder DDD-Modus zu inhibieren oder zu erzwingen, ferner in der Lage sind, nachdem sie ein Umschalten in den biventrikulären oder DDD-Modus erzwungen haben (26), die Rückkehr (38) in den Stimulationsmodus zu inhibieren, der die spontane atrioventrikuläre Leitung bevorzugt, solange die Vorrichtung einen Anstrengungszustand des Patienten detektiert.

8. Vorrichtung nach Anspruch 1, wobei die Mittel (20), um das bedingte Umschalten der Vorrichtung in den biventrikulären oder DDD-Modus zu inhibieren oder zu erzwingen:
- in der Lage sind, den aktuellen Wert der hämodynamischen Kennzahl (*Indice*_*EA*_*encours*) mit einer hämodynamischen Referenzkennzahl (*Indice*_*EA*_*ref*_*encours*) zu vergleichen (22); und
- in der Lage sind, das Umschalten der Vorrichtung in den biventrikulären oder DDD-Modus zu erzwingen (26), wenn der aktuelle Wert der hämodynamischen Kennzahl kleiner ist als die hämodynamische Referenzkennzahl.

9. Vorrichtung nach Anspruch 8, wobei:
- der Wert der hämodynamischen Referenzkennzahl *(EA_{ref}*) ein abhängig von der Herzfrequenz (*F_{c}*) variabler Wert ist, und
- die Vorrichtung Mittel zum dynamischen Erhöhen des Werts der hämodynamischen Referenzkennzahl umfasst, wenn sich die aktuelle Herzfrequenz erhöht.

10. Vorrichtung nach Anspruch 9, wobei:
- der Wert der hämodynamischen Referenzkennzahl (*EA_{ref}*) ein Wert ist, der zwischen den Mindest-(*Indice_EA_ref_repos*) und Höchstgrenzen (*Indice_EA_ref_exer*) variabel ist, und
- die Vorrichtung Mittel (42-62) umfasst, um die Mindest- und Höchstgrenzen dynamisch zu aktualisieren.

11. Vorrichtung nach Anspruch 8, wobei:
- die Vorrichtung Diagnosemittel umfasst, die in der Lage sind, das Auftreten eines permanenten atrioventrikulären Blocks zu bestimmen, und
- der Wert der hämodynamischen Referenzkennzahl (*EA_{ref}*) in Gegenwart eines permanenten atrioventrikulären Blocks, der von den Diagnosemitteln ermittelt wird, auf einen Wert gezwungen wird, der von der Herzfrequenz unabhängig ist.

12. Vorrichtung nach Anspruch 1, wobei der endokardiale Akzelerationssensor ein Sensor der Gruppe ist, gebildet aus: endokavitärem oder epikardialem Akzelerationssenor; Myokardwandbewegungssensor; intrakardialem Drucksensor; intrakardialem Bioimpedanzsensor; optischem Messsensor der Sauerstoffsättigung; Ultraschall-Messsensor der Volumenvariation.

13. Vorrichtung nach Anspruch 12, wobei der hämodynamische Sensor ein endokardialer Akzelerationssensor ist, der in der Lage ist, ein Signal (EA) zu senden, das für Bewegungen repräsentativ ist, die von den zyklischen Myokardkontraktionen erzeugt werden.

14. Vorrichtung nach Anspruch 13, wobei die Vorrichtung Mittel umfasst, die in der Lage sind, in dem Signal, das von dem Sensor gesendet wird, eine Komponente (EA₁) zu erkennen und zu isolieren, die einem endokardialen Akzelerationspeak entspricht, der mit der ventrikulären Kontraktion assoziiert ist, und die hämodynamische Kennzahl von der Amplitude dieser Komponente abzuleiten.

15. Vorrichtung nach Anspruch 13, wobei die Vorrichtung Mittel umfasst, die in der Lage sind, in dem Signal, das von dem Sensor gesendet wird, mindestens zwei Komponenten (T_{1EA}, T_{2EA}) zu erkennen und zu isolieren, die jeweils zwei endokardialen Akzelerationspeaks entsprechen, die mit der ventrikulären Kontraktion assoziiert sind, und die hämodynamische Kennzahl von einem Zeitintervall (Δ*Tx*₁) abzuleiten, das diese beiden Komponenten trennt.

## Claims

1. An active implantable medical device such as a cardiac prosthesis for stimulation, resynchronization and/or defibrillation, comprising:
- means for detecting spontaneous atrial and ventricular events;
- means for ventricular and atrial stimulation;
- means for operating the device in DDD or biventricular mode with ventricular detection and ventricular stimulation in the absence of spontaneous ventricular depolarization detected at the end of an atrioventricular delay (AVD); and
- mode switching means, for conditionally controlling, as a function of predetermined criteria, the conditional switching of the device towards the DDD or biventricular mode from a stimulation mode favouring the spontaneous atrioventricular conduction, and conversely the switching-back toward this latter mode from the DDD or biventricular mode,
the device being **characterized in that** it comprises:
- an hemodynamic sensor;
- means for deriving from the signal delivered by the hemodynamic sensor an hemodynamic index (*Indice_EA_encours*) representative of the tolerance of the patient to the spontaneous atrioventricular conduction ; and
- means (20) for inhibiting, or forcing, said conditional switching of the device towards the DDD or biventricular mode as a function of said hemodynamic index.

2. The device of claim 1, wherein the stimulation mode favouring the spontaneous atrioventricular conduction towards the DDD or biventricular mode is an AAI mode with ventricular detection.

3. The device of claim 1, wherein the stimulation mode favouring the spontaneous atrioventricular conduction towards the DDD or biventricular mode is a DDD or biventricular mode with hysteresis of the atrioventricular delay.

4. The device of claim 1, wherein:
- the device comprises diagnostic means (14) adapted to determine the occurrence of an atrioventricular block, and
- the means (20) for inhibiting, or forcing, the conditional switching of the device towards the DDD or biventricular mode are selectively activatable means, activated only in the absence of an atrioventricular block proved by the diagnostic means.

5. The device of claim 1, wherein:
- the device comprises means for evaluating the current value of the interval AR (*AR₁*, *AR₂*) separating an atrial stimulation (A) from the consecutive spontaneous ventricular depolarization (R);
- the device comprises means (18) for comparing the current value of the interval AR to a first threshold (*MaxARhemo*), and
- the means (20) for inhibiting, or forcing, the conditional switching of the device towards the DDD or biventricular mode are selectively activatable means, activated only if the current value of the interval AR is higher than the first threshold.

6. The device of claim 5, wherein:
- the first threshold (*MaxARhemo*) is a variable threshold, which is a function of the heart rate (Fc), and
- the device comprises means for dynamically reducing the value of the first threshold when the current heart rate increases.

7. The device of claim 1, wherein:
- the device further comprises means (36) for detecting a state of effort of the patient; and
- the means (20) for inhibiting, or forcing, the conditional switching of the device towards the DDD or biventricular mode are further adapted, after having forced (26) a switching towards the DDD or biventricular mode, to inhibit the switching-back (38) towards the stimulation mode favouring the spontaneous atrioventricular conduction as long as the device detects a state of effort of the patient.

8. The device of claim 1, wherein the means (20) for inhibiting, or forcing, the conditional switching towards the DDD or biventricular mode:
- are adapted to compare (22) the current value of the hemodynamic index (*Indice*_*EA*_*encours*) with a reference hemodynamic index (*Indice_EA_ref_encours*); and
- are adapted to force (26) the switching of the device towards the DDD or biventricular mode when the current value of the hemodynamic index is lower than the reference hemodynamic index.

9. The device of claim 8, wherein:
- the value of the reference hemodynamic index (*EA_{ref}*) is a value that varies as a function of the heart rate (Fc), and
- the device comprises means for increasing dynamically the value of the reference hemodynamic index when the current heart rate increases.

10. The device of claim 10, wherein:
- the value of the reference hemodynamic index (*EA_{ref}*) is a value that varies between minimum limit (*Indice_EA_ref_repos*) and a maximum limit (*Indice*_*EA*_*ref*_*exer*), and
- the device comprises means (42-62) for dynamically updating said minimum and maximum limits.

11. The device of claim 8, wherein:
- the device comprises diagnosis means adapted to determine the occurrence of a permanent atrioventricular block, and
- the value of the reference hemodynamic index (*EA_{ref}*) is forced to a value independent of the heart rate in presence of a permanent atrioventricular block proved by the diagnostic means.

12. The device of claim 1, wherein the endocardial acceleration sensor is a sensor of the group formed by: endocavitary or epicardial acceleration sensor; myocardium wall movement sensor; intracardiac pressure sensor; intracardiac bioimpedance sensor; optical oxygen saturation sensor; ultrasonic volume-variation measurement sensor.

13. The device of claim 12, wherein the hemodynamic sensor is an endocardial acceleration sensor, adapted to deliver a signal (EA) representative of the movements produced by the cyclic contractions of the myocardium.

14. The device of claim 13, wherein the device comprises means adapted to recognize and isolate in the signal delivered by the sensor a component (EA₁) corresponding to a peak of endocardial acceleration associated to the ventricular contraction, and to derivate said hemodynamic index from the amplitude of this component.

15. The device of claim 13, wherein the device comprises means adapted to recognize and isolate in the signal delivered by the sensor at least to components (T_{1EA}, T_{2EA}) corresponding to two respective peaks of endocardial acceleration associated to the ventricular contraction, and to derivate said hemodynamic index from a time interval (Δ*Tₓ*) separating these two components.
